# EUROPEAN PATENT APPLICATION

(11) **EP 0 900 786 A1**
(43) Date of publication of application: **10.03.1999**
(21) Application number: 97870130.8
(22) Date of filing: 03.09.1997
(51) Int. Cl.: C07C 317/44, C07C 315/02, A01N 57/26, A01N 25/30

(54) **New surface active compounds, method for their preparation, and their use**

(71) Applicant: MONSANTO EUROPE S.A./N.V., B-1150 Brussels (BE)
(72) Inventor: Pucci, Bernard, 13940 Molleges (FR); Barthelemy, Philippe, 84300 Cavaillon (FR); Polidori, Ange, Le St. Lazare, Bâtiment D, 84000 Avignon (FR); Lacombe, Jean Michel, 84700 Sorgues (FR); Toussaint, Marc Emile, 5872 Corroy-Le-Grand (BE); Bonnet, Marc René Edouard, 4000 Liège (BE)
(74) Representative: Bosch, Henry

(57) **Abstract**

Compound according to formula 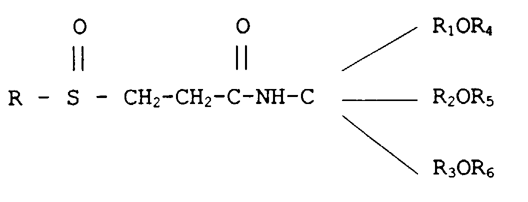 wherein
R is a linear or branched alkyl radical, optionally substituted having from 2 to 40 C-atoms; R₁, R₂, and R₃ are the same or different and selected from lower alkyl; and R₄, R₅, and R₆ are the same or different and selected from hydrogen, halogen or an alkali metal or alkali earth metal.
The compound shows interesting surface active properties and a low toxicity profile, and is suitable for cosmetic, detergent and agricultural compositions.

## Description

This invention relates to new surface active compounds and their use, more particularly in agrochemical compositions, especially herbicidal compositions, most preferably glyphosate compositions. The invention also relates to a method for the preparation of such compounds.

Several types of surfactants are already known in the art. There nevertheless remains a need for surfactants that show interesting surface tension properties while also showing a low toxicity profile, more particularly low eye or skin irritation properties and low fish toxicity properties, and other properties of significance for the environment. These environmental properties are becoming more and more important for additives whether they are used in the detergent field, in the cosmetic field or in the agricultural field or others.

FR-2 669 331 discloses a new class of non-ionic surface active compounds, such as N-tris(hydroxyalkyl)thioalkyl-amidomethane. Such surface active compounds are said to be suitable in cosmetic applications, detergent applications, chemical synthesis, as solubilisers or emulsifiers for use in agrochemical applications, pharmaceutical applications, and others; this document, however, while mentioning such agrochemical uses, does not actually disclose same.

It is well known that agrochemicals are combined with surface active materials that enhance their efficacy, more particularly with surfactants, either by mixing the relevant active ingredient in the farmer's tank with one or more surfactants at appropriate rates or by formulating ready-mixes that are concentrates of the relevant active ingredient with one or more surfactants. Numerous studies have been made on the effect of additives on the herbicidal effect of herbicides, for instance, and more particularly on the action of glyphosate. For example Wyrill and Burnside, Weed Science, Vol. 25 (1977), 275-287, examined solutions containing different classes of surfactant. Some classes of surfactant were more effective than others in enhancing the herbicidal effect of glyphosate (used as a solution of the isopropylamine salt), but Wyrill and Burnside concluded that an effective surfactant is a critical component of any glyphosate spray mixture.

European patent No. 0 206 537 discloses the use of Emcol-CC 57, a polypropoxylated quaternary ammonium surfactant, in solid phytoactive glyphosate compositions. EP-A-0 441 764 discloses herbicidal glyphosate compositions comprising a polypropoxylated quaternary ammonium surfactant. The compositions disclosed are said to be herbicidally effective to a similar extent as were glyphosate compositions comprising ethoxylated fatty amine surfactant considered to be optimum, and in addition, such compositions are said to have a low toxicity to fish and to be non-irritant (to skin and eyes).

The present invention provides for new compounds having the formula (I) 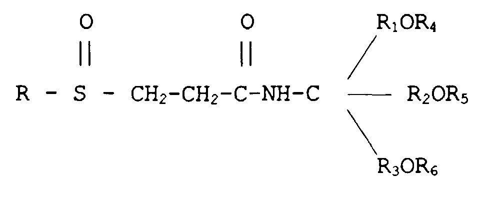 wherein
R is a linear or branched alkyl radical, optionally substituted having from 2 to 40 C-atoms; R₁, R₂, and R₃ are the same or different and selected from lower alkyl; and R₄, R₅, and R₆ are the same or different and selected from hydrogen, halogen or an alkali metal or alkali earth metal.

In the above formula, R represents the hydrophobic or lipophilic part of the molecule, and the opposite part of the molecule, which is the hydrophilic part thereof, can be designed in the light of the applications of the compound by proper selection of R₁, R₂, R₃, R₄, R₅ and R₆. In the case of the use as a surface active additive, it is preferred that R₁, R₂, and R₃ are all methylene.

In a preferred embodiment, R is a linear alkyl radical having from 6 - 20 C-atoms, most preferably 8 - 16 C-atoms.

Such new surface active compounds which have a hydrocarbon tail, a non-ionic polar head and a sulfoxide moiety may be used in cosmetics, as detergents, in pharmaceutical formulations and in agrochemical formulations.

It has been found that the compounds of the invention show particularly interesting low-toxicity properties as will be explained in more detail below.

Further, it has been found that the invention compounds are particularly suitable as additives for agrochemical compositions, more particularly herbicidal formulations, especially herbicidal glyphosate formulations. As will be seen further below, the invention compounds appear to significantly enhance the herbicidal activity of glyphosate herbicide, while showing an interesting toxicity profile. They can advantageously be used as a constituent, preferably the major constituent, of the surface active component of agricultural compositions, preferably herbicidal compositions, most preferably glyphosate compositions.

As a result, it is now possible to prepare herbicidal glyphosate formulations that have an environmental profile similar to the one of the formulations disclosed in EP-A-0 441 764, while being at least as efficient as same.

Herein, the term "glyphosate herbicide" means N-phosphonomethylglycine (glyphosate) and any form or derivative of glyphosate which in aqueous solution provides glyphosate anions. Suitable cations may also be present. Examples of such suitable cations are alkali metal cations, for instance sodium and potassium, and ammonium, diammonium and substituted ammonium cations. The latter include cations derived from primary or secondary amines such as isopropylamine or dimethylamine, and from diamines such as ethylenediamine.

Other examples of agriculturally acceptable salts of glyphosate are the trimethyl sulfonium salt of glyphosate, or aminoguanidine salts as disclosed in EP-A-0 088 180. Because glyphosate has more than one replaceable hydrogen atom, mono- and di-alkali metal salts are possible, as well as mixtures of such salts. In the herbicidal compositions of this invention,

the weight ratio of glyphosate (expressed as glyphosate acid equivalent) to the surface active component can vary over a considerable range, for example from about 1:5 to about 10:1. The optimum ratio will vary according to the manner in which the herbicidal composition is applied, the weed species to be treated, and the particular quaternary ammonium compound selected, but is normally within the range from about 1:2 to about 5:1, for example about 1:1, about 2:1, or about 4:1. The invention compound preferably constitutes up to 100% of the surface active component.

A composition of the invention can be a liquid aqueous concentrate intended to be diluted with water to form a spray solution for the actual herbicidal application. A liquid concentrate will normally contain at least 50 grams glyphosate acid equivalent per litre, and preferably at least 100 g/l. Compositions of the invention may include a significant amount of an agriculturally-acceptable inorganic ammonium salt such as ammonium sulphate, in addition to the glyphosate and the invention compound. Liquid concentrates without such an inorganic ammonium salt can contain up to 450 g/l or more, for example 300-450 g/l, glyphosate acid equivalent when the glyphosate is present as a salt having a high solubility, for example the isopropylamine salt. In liquid concentrates containing an inorganic ammonium salt (for example ammonium sulphate in an amount of from 100 to 500 g/l), the maximum amount of glyphosate which can be accommodated is less, and may be, for example, about 150 g/l glyphosate acid equivalent. Such liquid concentrate may require, depending on the loading, the presence of a compatibility agent, which the man skilled in the art will find without undue burden of experimentation.

In another form, the composition of the invention is a solid, for example a free-flowing particulate, granular solid or compressed into tablets or briquets of any desired size and shape. The term "solid" as employed herein includes granular, particular, wettable powder, water soluble and water dispersible formulations, or mixtures thereof. Typically such solids are dry. Such dry compositions will usually contain not more than 5% and preferably not more than 1% by weight of water.

In a solid composition, glyphosate is preferably present as an alkali metal salt such as a sodium or potassium salt, or as an ammonium salt and in some cases the isopropylamine salt. The surfactant used in the compositions of the invention particularly allows for relatively high concentrations of glyphosate herbicide in the solid formulation.

Optionally solid compositions may be formulated to include a water-soluble inert carrier, and for this purpose ammonium sulphate, ammonium thiocyanate, or ammonium phosphate are particularly suitable. The weight ratio of glyphosate (expressed as glyphosate acid equivalent) to the surface active component in such compositions will be within the general range mentioned above. The amount of water-soluble inert carrier is not critical, and in the case of ammonium sulphate, the amount may, for example, range from 20% to 80% of the total weight of the composition. Solid compositions can be made, for example by spray drying an aqueous solution of the components, by dry-blending the ingredients in conventional blending apparatus, or by extrusion blending whereby a granular product is obtained in an essentially single operation. In a preferred method, an alkali metal or ammonium salt of glyphosate is produced in situ in an extruder as known in the art. It has been found that the product obtained by radial extrusion has better dissolution characteristics in water compared to a product obtained by frontal extrusion.

The compositions of the invention can be diluted as spray solutions which may be applied by spraying for example. In these solutions, the concentration of glyphosate is selected according to the volume per unit area of spray solution to be used and the desired rate of application of glyphosate per unit area. For example, conventional spraying is done at 100-600 litres of spray solution per hectare, and the rate of application of glyphosate is typically 0.125 to 6 kg of glyphosate acid equivalent per hectare, depending on the weed target. In controlled drop spraying, the rate of application of glyphosate per hectare will normally be in the same range, but the volume of spray solution per hectare will be considerably less, perhaps 15-50 litres per hectare. Spray solutions for controlled drop spraying are therefore more concentrated than those used in conventional spraying. In spray solutions containing inorganic ammonium salts in addition to the glyphosate and the invention compound, the amount of inorganic ammonium salt which can be included may be, for example, up to 10 times the weight of glyphosate acid equivalent.

Spray solutions can be prepared by diluting liquid concentrates with water or dissolving solid compositions in water as described above, or by tank mixing the separate constituents of the compositions.

Compositions containing the invention compound can optionally contain other constituents, preferably those and in amounts that do not have a substantial adverse effect on the resulting composition as to irritancy and toxicity. These additional constituents may include anti-freeze agents such as ethylene glycol, polyethylene or polypropylene glycols and/or glycerol. Other examples of additional components are dyes, thickening agents, anti-foam agents, for instance silicone-based anti-foam agents, agents suitable for pH adjustment to optimize herbicidal efficacy and compatibility or stability of the composition, and certain surfactants, for instance non-ionic surfactants such as polyoxyethylene ethers or esters, sugar ethers, ethoxylated alkylamine surfactants, quaternary ammonium compounds (such as disclosed in EP-0 441 764 or other commercially available ethoxylated and/or propoxylated quaternary ammonium salts as sold under the tradenames Ethoquad, Emcol) and sorbitan esters. Such compositions may also comprise one or more additional herbicides, themselves preferably having low or no irritancy and relatively low toxicity.

The surfactants as disclosed herein can also be used to prepare tank mix adjuvants for glyphosate spray dilutions, that may additionally comprise anti-freeze agents, dyes, thikening agents, anti-foam agents and/or co-surfactants.

According to another aspect of the present invention, there is provided a process for the manufacture of the invention compounds. Said compounds may be prepared by reaction of a corresponding alkanethiol with tris(hydroxyalkyl)acrylamidomethane (THAM), preferably in alcoholic medium, such as methanol, and in the presence of sodium methoxide, crystallisation, followed by oxidation and recrystallisation. Oxidation is advantageously carried out in the presence of hydrogen peroxide, and a catalytic quantity of sodium methylate, at a pH between 8 and 9, at elevated temperature.
As will be noted, the above process makes use of THAM as strating or intermediate material. While THAM is commercially available, it may also be manufactured according to the following process, which yields THAM at ligh yields and a high purity grade. The process comprises the reaction of tris (hydroxyalkyl) aminomethane with acryloyl chloride at reduced temperature and at a pH controlled between 7.5 and 8.5, precipitation and crystallisation.

### Example 1

### Preparation of Tris hydroxymethyl-(3-decylsulfinylpropanamido)-methane (6)

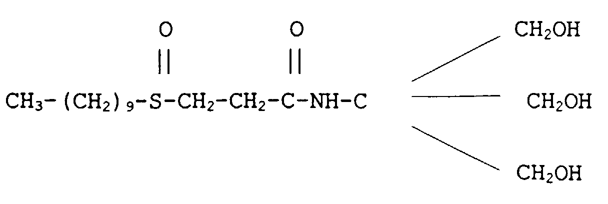

28.6 g (0.164 mole, 1 equivalent) of decanethiol were dissolved in 500 ml methanol. 14 g of sodium methoxide were added to the mixture. The solution was stirred for 3 minutes, at room temperature.
27 g (0.164 mole, 1 equivalent) of tris hydroxymethyl acrylamidomethane (THAM) were added in several fractions to the methanolic suspension. The mixture was then stirred for 3 hours at 60°C, and then allowed to cool at ambiant temperature. The solution is then neutralized at pH 6-7 with concentrated hydrochloric acid (12N) at 0° C. The methanol was evaporated under reduced pressure. The crude product was solubilized in hot ethyl acetate, filtered while hot and subsequently crystallised at room temperature. After filtration, the white powder obtained was triturated in hexane. The hexane was separated under vacuum. 38.1 g of a white precipitate (tris hydroxymethyl-(4-thiatetradecanamido)methane) were isolated; Yield:71.3%; Melting point:87 - 90°C.
NMR 1H (CDCL₃): δ in ppm 0.85 (t, 3H, CH₃CH₂); 1.3 (m, 14H, CH₂)₇); 1.6 (m, 2H, CH₂CH₂S); 2.5 (t, 4H, CH₂SCH₂); 2.8 (t, 2H, CH₂CO); 3.6 (s, 6H, C(CH₂)₃); 5.2 (m, 3H, OH); 6.7 (s, 1H, NH).
20 g (0.0576 mole, 1 equivalent) of the isolated compound were suspended in 150 ml methanol, in the presence of a catalytic amount of sodium methoxide (pH 8-9). 21.76 ml (0.0576 mole, 1 equivalent) hydrogen peroxide at 9% were added at ambient temperature. The reaction mixture was then heated at 60°C for 3 hours. The solvent was evaporated under reduced pressure. Residual water was coevaporated twice under vacuum with acetone. Recrystallisation in ethyl acetate yielded 18.15 g of a white powder; Yield: 86.3%; melting point: 66 - 67.5 °C.
Rf=0.5 (CH₃OH 1/CH₃COOCH₂CH₃9)
Formula=C₁₇H₃₅NO₅S
MW=365g/mole
*NMR 1H (CDCl*_{*3*}*)*: δ in ppm 0.9 (t, 3H, CH₃CH₂); 1.3 (m, 14H, (CH₂)₇); 1.8 (m, 2H, CH₂CH₂SO); 2.8 (m, 4H, CH₂SOCH₂); 3.1 (m, 2H, CH₂CO); 3.7 (q, 6H, C(CH₂)₃); 4.5 (m, 3H, OH); 7.1 (s, 1H, NH). *NMR*^{*13*}*C(CDCl*_{*3*}*)*: δ in ppm 14.74 (CH₃); 23.30; 29.47; 29.84; 29.92; 30.02; 32.15; 32.51; 47.64 (CH₂SO); 53.35 (CH₂SO); 62.85 (C(CH₂)₃); 64.13 (CH₂OH); 172.69 (CO).
*Mass spectrometry FAB*^{*+*} *(NBA)*: m/e = 388 (M+Na)⁺; m/e = 366 ((M+H)⁺; m/e = 176 (M-190+H)⁺; m/e = 158(176-18)⁺.
*Solubilizing properties*: (Percentage of proteins solibilized in the lyophilised subcellular fractions) membranes: 57.41% (+/-2.87); mitochondries: 70.95% (+/-7.24); microsomes: 68.90% (+/-5.67).

### Example 2

### Preparation of Tris hydroxymethyl-(3-dodecylsulfinylpropanamido)- methane (II)

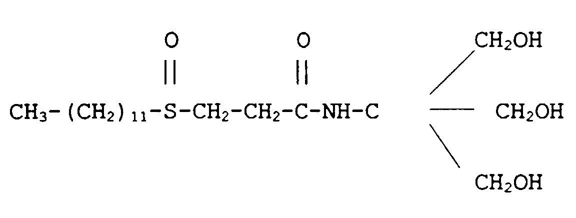

The same procedure as described in relation with Example 1 was followed: 50 g (0.242 mole, 1 equivalent) of dodecanethiol were reacted with 42 g (0.24 mole, 1 equivalent) THAM in the presence of 17 g sodium methoxide in 500 ml methanol. 49.7 g of a white precipitate were isolated (Yield:55%); melting point: 93-96° C.
*NMR 1H(CDCl*_{*3*}*)*: δ in ppm 0.85 (t, 3H, CH₃CH₂); 1.3 (m, 18H, (CH₂)₉); 1.6 (m, 2H, CH₂CH₂S); 2.5 (t, 4H, CH₂SCH₂); 2.8 (t, 2H, C(CH₂)CO); 3.6 (s, 6H, C(CH₂)₃); 5.2 (m, 3H, OH); 6.7.(s,1H, NH)
20 g (0.053 mole, 1 equivalent) of the isolated product were then reacted with 23.02 ml hydrogen peroxide at 9% (0.06 mole, 1.15 equivalent) in 150 ml methanol. 17.77 g of a white powder (II) were isolated; melting point 74° - 76°C (yield: 85.3%).
Rf=0.7 (CH₃OH2/CH₃COOCH₂CH₃8)
Formula = C₁₉ H₃₉ NO₅S
Mw = 393 g/mole
*NMR 1H(CDCl*_{*3*}*)*: δ in ppm 0.9 (t, 3H, CH₃CH₂); 1.4 (m, 18H, (CH₂)₉); 1.8 (m, 2H, CH₂CH₂SO); 2.8 (m, 6H, CH₂SOCH₂CH₂); 3.7 (s, 6H, C(CH₂)₃); 5 (s, 3H, OH); 7.1 (s, 1H, NH).
*NMR*^{*13*}*C(CDCl*_{*3*}*)*: δ in ppm 14.70 (CH₃); 23.28; 29.46; 29.84; 29.94; 30.02; 30.18; 30.23; 32.51; 47.56 (CH₂SO); 53.19 (CH₂SO); 63.02 (C(CH₂)₃); 63.41 (CH₂OH); 172.70 (CO).
*Mass spectrometry FAB*^{*+*} *(NBA)*: m/e = 416 (M+Na)⁺; m/e = 394 ((M+H)⁺; m/e = 176 (M-218+H)⁺; m/e = 158(176-18)⁺.
*Solubilizing properties*: (Percentage of proteins solubilized in the lyophilised subcellular fractions) membranes: 52.41% (+/-4.25); mitochondries: 65.62% (+/-4.68); microsomes: 62.47% (+/-4.10).

### Example 3

### Preparation of Tris hydroxymethyl-(3-hexadecylsulfinylpropanamido)- methane (III)

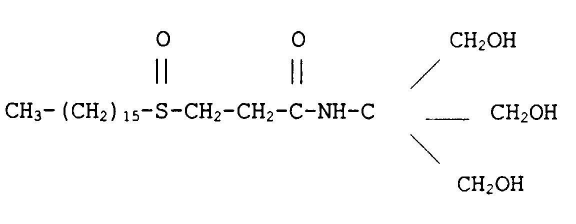

The same procedure as described in relation with Example 1 was followed: 24 g (0.093 mole, 1 equivalent) of hexadecanethiol were reacted with 17.9 g (0.1 mole, 1.07 equivalent) THAM in the presence of 6.8 g of sodium methoxide in 400 ml methanol. 31 g of a white precipitate were isolated (Yield:84.2%; melting point 98-100°C).
NMR 1H(CDCl₃): δ in ppm 0.85 (t, 3H, CH₃CH₂); 1.3 (m, 26H, (CH₂)₁₃); 1.6 (m, 2H, CH₂CH₂S); 2.5 (t, 4H, CH₂SCH₂); 2.8 (t, 2H, CH₂CO); 3.6 (s, 6H, C(CH₂)₃); 5.2 (m, 3H, OH); 6.7 (s, 1H, NH).
12.5 g (0.0288 mole, 1 equivalent) of the isolated product were subsequently reacted with 11.96 ml hydrogen peroxide at 9% (0.0316 mole, 1.1 equivalent) in 150 ml methanol. 9.6 g of a white powder (III) were isolated (yield:74.1%; melting point 77-79°C).
Rf=0.6 (CH₃OH 1/CH₃COOCH₂CH₃9)
Formula = C₂₃H₄₇NO₅S
Mw=449 g/mole
*NMR 1H(CDCl*_{*3*}*)*: δ in ppm 0.9 (t, 3H, CH₃CH₂); 1.4 (m, 26H, (CH₂)₁₃); 1.8 (m, 2H, CH₂CH₂SO); 2.8 (m, 6H, CH₂SOCH₂CH₂); 3.7 (s, 6H, C(CH₂)₃); 4.5 (s, 3H, OH); 7.1 (s, 1H, NH).
*NMR*^{*13*} *C(CDCl*_{*3*}*)*: δ in ppm 14.75 (CH₃); 23.32; 27.61; 29.46; 29.85; 30.02; 30.33; 32.58; 47.64 (CH₂SO); 53.38 (CH₂SO); 63.02 (C(CH₂)₃); 64.55 (CH₂OH); 172.73 (CO).
*Mass spectrometry FAB*^{*+*} *(NBA)*: m/e = 472 (M+Na)⁺; m/e = 450 ((M+H)⁺; m/e = 176 (M-274+H)⁺; m/e = 158(176-18)⁺.
*Solubilizing properties*: (Percentage of proteins solubilized in the lyophilised subcellular fractions) membranes: 47.71% (+/-2.87); mitochondries: 58.64% (+/-5.78); microsomes: 58.41% (+/-3.14).

### Example 4 surface active properties

The CMC (critical micellar concentration) was measured with a Krüss tensiometer known in the art. The following results were obtained:

| Cpd where **n** is | **CMC (mM/l)** | **CMC (mg/l)** | **γ(mN.m**^{**-1**}**)** |
|---|---|---|---|
| 7 | 0.74 | 250 | 25.5 |
| 9 | 0.34 | 124 | 26 |
| 11 | 0.064 | 25 | 29 |
| 15 | 0.015 | 6.8 | 34.5 |

### Example 5

A composition comprising 19.51 g of glyphosate isopropylamine salt (352.0 g/l acid equivalent), 4.5 g of a surfactant component, and 5.99 g demineralised water was prepared according to procedures known to the person skilled in the art. The surfactant component was comprised of 50% w/w of (I) and 50% w/w of a surfactant mix comprising 80% of a quaternary ammonium surfactant according to EP-0 441 764 (wherein R1=R2=R3=CH3 and n=8) and 20% of ethoxylated sorbitan monolaurate. This composition has been bioevaluated as appears from the examples below.

### Example 6

A further concentrate formulation having the following composition was prepared:
- 17.74 g (59.15%w/w) glyphosate IPA-salt,
- 4.09 g (13.64% w/w) of compound (I),
- 8.16 g (27.21% w/w) demin. water.
This formulation has been tested below.

### Examples 7 and 8

The following compositions were prepared and tested:
- Example 7:: - 19.51 g (65.03 %w/w of glyphosate IPA-salt;
- 2.25 g (7.50 %w/w) of quat ammonium surfactant as in Example 5;
- 2.25 g (7.50 %w/w) of compound (II);
- 5.99 g (19.97 %w/w) demin. Water.
- Example 8:: - 17.74 g (59.15%w/w) glyphosate IPA-salt,
- 4.09 g (13.64% w/w) of compound (II),
- 8.16 g (27.21% w/w) demin. Water.
Both these formulations were tested as follows.

### Example 9

The compositions of Examples 5 to 8 were evaluated in a greenhouse trial. Such compositions were compared with the commercial formulation Roundup® (360 g/l a.e. as the isopropylamine salt of glyphosate and 180g/l ethoxylated tallow amine surfactant), referred to as Rup1, and with a commercial formulation of the isopropylamine salt of glyphosate with a surfactant component (in a ratio of 2/1) which itself consists in a mixture of a quaternary ammonium surfactant according to EP-A-0 441 764 (wherein R¹=R²=R³=CH₃ and n=8) with ethoxylated sorbitan monolaurate, referred to as Rup2. Glyphosate herbicide was applied at the rates indicated.

The following species, *Ipomea purpureum, Raphanus sativus, and Agropyron repens* were grown from seed in a growth-room according to good agronomical practice. All plants were grown under 50% and 75% RH during the night and the day, respectively.

Before spraying, pots were selected for uniformity as far as possible, and atypical examples were discarded. Spray solutions were applied with an automatic spraying cabinet reproducing field spraying conditions, calibrated to deliver spray solution at a rate equivalent to 200 l/ha with Teejet nozzles 8002. All replicate pots (3-5 replicates per species, per treatment) were sprayed with one pass of the sprayer.

After spraying, the plants were transferred to a greenhouse (about 20°C) and control pots were placed at random among treated pots.

Assessment of "% phytotoxicity" were made by comparison with untreated controls on an arbitrary scale from 0 to 100%, where 0 means no visible effect and 100% means death of all plants.

Assessments were made at interval is (DAT : days after treatment) as indicated. The results are shown in the following Table of results.

**TABLE I**

| **% phytotoxicity at 34 DAT** | | | | |
|---|---|---|---|---|
| **Treatments** | **Rate g ae/HA** | ***Ipomea** **purpurea*** | ***Raphanus** **sativus*** | ***Agropyron** **repens*** |
| RUP 1 | 250 | 33 | 45 | 44 |
| RUP 1 | 500 | 49 | 58 | 63 |
| RUP 1 | 750 | 58 | 53 | 78 |
| RUP 1 | 1000 | 90 | 75 | 79 |
| RUP 2 | 250 | 31 | 28 | 39 |
| RUP 2 | 500 | 34 | 40 | 59 |
| RUP 2 | 750 | 44 | 58 | 78 |
| RUP 2 | 1000 | 73 | 78 | 89 |
| EX. 5 | 250 | 29 | 34 | 48 |
| EX. 5 | 500 | 43 | 44 | 73 |
| EX. 5 | 750 | 55 | 59 | 81 |
| EX. 5 | 1000 | 76 | 66 | 91 |
| EX. 6 | 250 | 33 | 28 | 41 |
| EX. 6 | 500 | 39 | 48 | 59 |
| EX. 6 | 750 | 46 | 61 | 75 |
| EX. 6 | 1000 | 73 | 68 | 89 |

**TABLE II**

| **% phytotoxicity at 27 DAT** | | | | |
|---|---|---|---|---|
| **Treatments** | **Rate g ae/HA** | ***Ipomea** **purpurea*** | ***Raphanus** **sativus*** | ***Agropyron** **repens*** |
| RUP 1 | 300 | 48 | 64 | 81 |
| RUP 1 | 600 | 69 | 84 | 93 |
| RUP 1 | 900 | 81 | 74 | 100 |
| RUP 1 | 1200 | 84 | 83 | 94 |
| RUP 2 | 300 | 25 | 53 | 76 |
| RUP 2 | 600 | 33 | 66 | 98 |
| RUP 2 | 900 | 65 | 85 | 95 |
| RUP 2 | 1200 | 85 | 89 | 100 |
| EX. 5 | 300 | 45 | 53 | 84 |
| EX. 5 | 600 | 59 | 71 | 96 |
| EX. 5 | 900 | 70 | 75 | 100 |
| EX. 5 | 1200 | 76 | 83 | 99 |
| EX. 6 | 300 | 40 | 53 | 70 |
| EX. 6 | 600 | 58 | 74 | 98 |
| EX. 6 | 900 | 61 | 69 | 100 |
| EX. 6 | 1200 | 61 | 73 | 94 |
| EX. 7 | 300 | 35 | 44 | 68 |
| EX. 7 | 600 | 50 | 63 | 91 |
| EX. 7 | 900 | 53 | 83 | 98 |
| EX. 7 | 1200 | 84 | 91 | 99 |
| EX. 8 | 300 | 23 | 35 | 55 |
| EX. 8 | 600 | 51 | 64 | 96 |
| EX. 8 | 900 | 39 | 69 | 91 |
| EX. 8 | 1200 | 54 | 81 | 93 |

As can be seen from the data, the surfactants according to the invention enhance the herbicidal activity of glyphosate to a similar extend as the surfactants of commercial glyphosate formulations, while showing a good environmental profile.

### Example 10

### Preparation of Tris(hydroxymethyl)acrylamidomethane.

61.5 g (0.5 Mole, 1eq) of tris(hydroxymethyl)acrylamido methane were dissolved in 400 ml methanol at 40°C in a three necked flask; the solution was cooled at 0-5°C. 67,87 g (0.75 Mole, 1.5eq) of acryloyl chloride were added drop-wise to the well stirred solution. The pH was controlled and maintained within a range of 7.5 to 8.5 by adding a 3N methanolic solution of potassium hydroxide. Thereafter, the solution was stirred at 5-10°C for 2 hours, then acidified at pH=3 with a 3N sulfuric acid solution. Salts were filtered off and the solvent evaporated under reduced pressure. The tris(hydroxymethyl)acrylamidomethane precipitate was dissolved in hot ethanol, the residual salts were filtered. After crystallization at 0°C, 55 g of a white precipitate were isolated. After concentration, another crop of THAM (15 g) was obtained. Global yield = 80%. Mp = 142°C.

## Claims

1. Compound according to formula 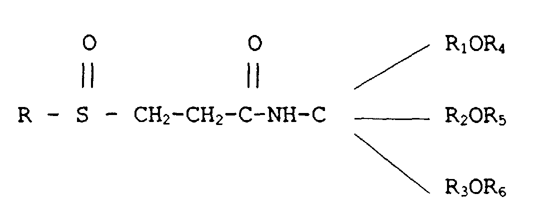 wherein
R is a linear or branched alkyl radical, optionally substituted having from 2 to 40 C-atoms; R₁, R₂, and R₃ are the same or different and selected from lower alkyl; and R₄, R₅, and R₆ are the same or different and selected from hydrogen, halogen or an alkali metal or alkali earth metal.

2. Compound according to Claim 1 wherein R₁, R₂, and R₃ are all methylen.

3. Compound according to Claim or 2 wherein R₄, R₅ and R₆ are all hydrogen.

4. Compound according to any of Claims 1 to 3 wherein R is a linear alkyl chain having from 6 to 20 C-atoms, preferably 8 to 16 C-atoms.

5. Process for the preparation of compounds according to any of the preceding Claims characterized in that a corresponding alkanethiol is reacted with tris(hydroxyalkyl)acrylamidomethane, preferably in alcoholic medium, such as methanol, and in the presence of sodium methoxide, the product obtained is crystallized, and subsequently oxidized and then recrystallized.

6. Process according to Claim 5 characterized in that the oxidation is carried out in the presence of hydrogen peroxide, and a catalytic quantity of sodium methoxide, at a pH between 8 and 9, at elevated temperature.

7. Agricultural composition comprising an agriculturally effective active material and a surface active component which comprises a compound according to any of Claims 1 to 4.

8. Agricultural composition according to Claim 6 characterized in that the agriculturally effective active material is a herbicide, more especially glyphosate herbicide in its acid form or as derivatives thereof.

9. Agricultural composition according to Claim 7 characterized in that the weight ratio of glyphosate (expressed as glyphosate acid equivalent) to the surface active component is comprised between 1:5 and 10:1, preferably 1:2 to 5:1, most preferably about 2:1.

10. Adjuvant for agricultural compositions characterized in that it comprises a compound according to any of Claims 1 to 4 together with anti-freeze agents, such as ethylene glycol, polyethylene or polypropylene glycols and/or glycerol, dyes, thickening agents, anti-foam agents, for instance silicone-based anti-foam agents, agents suitable for pH adjustment, and certain surfactants, for instance non-ionic surfactants such as polyoxyethylene ethers or esters, sugar ethers, ethoxylated alkylamine surfactants, quaternary ammonium compounds (such as disclosed in EP-0 441 764 or other commercially available ethoxylated and/or propoxylated quaternary ammonium salts as sold under the tradenames Ethoquad, Emcol) and sorbitan esters.

11. Use of agricultural compositions according to any of Claims 6 to 8 for the control of pest.

12. Process for the preparation of tris(hydroxyalkyl) amidomethane comprising the reaction, preferably in alkanolic medium, more preferably methanol, of tris(hydroxyalkyl)aminomethane with acryloyl chloride at reduced temperature and at a pH controlled between 7.5 and 8.5, preferably by addition of a methanolic solution of potassium hydroxide, precipitation and separation, and crystallisation.
